# EUROPEAN PATENT APPLICATION

(11) **EP 4 589 007 A1**
(43) Date of publication of application: **23.07.2025**
(21) Application number: 24152487.5
(22) Date of filing: 17.01.2024
(51) Int. Cl.: C12N 15/11, C12N 15/63, C12N 15/864, C12N 15/867

(54) **PRODUCTION OF VIRAL VECTORS**

(71) Applicant: Revvity Gene Delivery GmbH, 82166 Gräfelfing (DE)
(72) Inventor: Salomon, Michael, 82288 Kottgeisering (DE); Thirion, Christian, 80337 München (DE)
(74) Representative: Dentons Patent Solutions Rechtsanwaltsgesellschaft mbH

(57) **Abstract**

The present disclosure relates to a combination of nucleic acids for the production of viral particles, said combination comprising or consisting of (a) a first nucleic acid encoding or being an inhibitory RNA; (b) at least one second nucleic acid comprising helper nucleic acids necessary for production of said viral particles, and/or encoding helper proteins necessary for said production; and (c) a third nucleic acid comprising a binding site for said inhibitory RNA. Furthermore, provided are methods of transfecting a cell with said combination, a production cell obtained by said transfecting or comprising the components of said combination and methods of producing viral particles.

## Description

### BACKGROUND

The present disclosure provides a combination of nucleic acids for the production of viral particles, said combination comprising or consisting of (a) a first nucleic acid encoding or being an inhibitory RNA; (b) at least one second nucleic acid comprising helper nucleic acids necessary for production of said viral particles, and/or encoding helper proteins necessary for said production; and (c) a third nucleic acid comprising a binding site for said inhibitory RNA.

In this specification, a number of documents including patent applications and manufacturer's manuals are cited. The disclosure of these documents, while not considered relevant for the patentability of this disclosure, is herewith incorporated by reference in its entirety. More specifically, all referenced documents are incorporated by reference to the same extent as if each individual document was specifically and individually indicated to be incorporated by reference.

Many genetic engineering applications in research, diagnosis and therapy involve the transfer of foreign DNA to target cells, tissues, or organisms. Viral vectors or particles, such as adeno-associated viral particles or retroviral, including lentiviral particles comprising foreign DNA are useful tools for this purpose.

A frequent problem in the production of viral vectors comprising a nucleic acid encoding a protein of interest is that, during said production in a production cell line, significant amounts of said protein are produced, which in turn consume nutrients and have adverse effects on the production cell. This may deteriorate viral particle yield, abolish yield altogether or cause cell death.

The present disclosure addresses this problem. A technical problem underlying the instant disclosure may be seen as the avoidance of such adverse effects, such as improving viral particle yield and avoiding cell death and/or the provision of improved means and methods for the production of viral vectors encoding proteins of interest.

This problem has been solved by the aspects and embodiments disclosed below and as shown in the Examples.

### SUMMARY

In a first aspect, the present disclosure provides a combination of nucleic acids for the production of viral particles, said combination comprising or consisting of (a) a first nucleic acid encoding or being an inhibitory RNA; (b) at least one second nucleic acid comprising helper nucleic acids necessary for production of said viral particles, and/or encoding helper proteins necessary for said production; and (c) a third nucleic acid comprising a binding site for said inhibitory RNA.

In a second aspect, the present disclosure relates to the use of the combination of the first aspect for transfection of a cell.

In a third aspect, the present disclosure provides a method of transfecting a cell, said method comprising bringing a cell into contact with the components of the combination of the first aspect.

In a fourth aspect, the present disclosure provides a cell or a cell line (a) obtained by the method of the third aspect; and/or (b) comprising the components of the combination of the first aspect.

In a fifth aspect, the present disclosure provides a method of producing viral particles carrying a nucleic acid of interest, said method comprising (a) bringing a cell into contact with the components of the combination of the first aspect, more specifically those embodiments of the first aspect which require presence of a nucleic acid of interest; and (b) allowing formation of said viral particles to occur.

### BRIEF DESCRIPTION OF THE DRAWINGS

**Figure 1** shows the plasmid map of pAAV-CMV-eGFP-WPRE-IBD1 (transgene) plasmid.
**Figure 2** shows the plasmid map of pAAV-EFS-mRIPK3-IBD1 (transgene) plasmid.
**Figure 3** shows the plasmid map of pAAV-EFS-TRIF-IBD1 (transgene) plasmid.
**Figure 4** shows the plasmid map of pAAV-EFS-TRIF-T2a-mRIPK3-IBD1 (transgene) plasmid.
**Figure 5** shows the plasmid map of pAAV-CMV-eGFP-KASH-IBD1 (transgene) plasmid.
**Figure 6** shows the plasmid map of pAAV-EFS-mRIPK3-IBD2 (transgene) plasmid.
**Figure 7** shows the plasmid map of pAAV-EFS-mRIPK3-IBD3 (transgene) plasmid.Figure 8 shows the plasmid map of pHelper-U6shRNA1 (pHelper) plasmid.
**Figure 9** shows the plasmid map of pHelper-H1-shmir1 (pHelper) plasmid.
**Figure 10** shows the plasmid map of pRep2-CAP2 (packaging) plasmid.
**Figure 11** shows brightfield and fluorescence pictures of (A) transfection of pRep2-CAP2 + pHelper-U6shRNA1 + pAAV-CMV-eGFP-WPRE-IBD1; (B) control transfection without the shRNA binding site: pRep2-CAP2 + pHelper-U6shRNA1 + pAAV-CMV-eGFP and (C) control transfection without shRNA1: pRep2-CAP2 + pHelper + pAAV-CMV-eGFP-WPRE-IBD1.
**Figure 12** shows a chart representing the vector genome titers as fold increase in vg, measured by qPCR for rAAV productions of various serotypes with and without RNAi mediated suppression of eGFP expression during rAAV production.
**Figure 13** shows a chart representing the vector genome titers as fold increase in vg, measured by qPCR for rAAV production of AAV2-7m8 serotype with and without RNAi mediated suppression of mRIPK3, TRIF, T2a-mRIPK3 and eGFP-KASH expression during rAAV production.
**Figure 14** shows a chart representing the vector genome titers as fold increase in vg, measured by qPCR for rAAV productions of of AAV2-7m8 serotype with and without shRNA and shmir-mediated suppression of mRIPK3 expression during rAAV production.

### DETAILED DESCRIPTION

Given that the first aspect requires a plurality of nucleic acids, it is also referred to as "combination". Said combination may also be viewed as a "system" or a "kit". Of note, the components of the combination may also be distributed over more than one actual system or kit, wherein in case of such a plurality of systems or kits, it is the sum of all systems or kits which provides all components of the combination.

Throughout this specification, the word "comprise" or variations, such as "comprises" or "comprising" will be understood to imply the inclusion of a stated integer (or components) or group of integers (or components), but not the exclusion of any other integer (or components) or group of integers (or components).

Throughout the specification, where compositions or combinations are described as having, including, or comprising (or variations thereof), specific components, it is contemplated that compositions also may consist essentially of, or consist of, the recited components. Similarly, where methods or processes are described as having, including, or comprising specific process steps, the processes also may consist essentially of, or consist of, the recited processing steps. Further, it should be understood that the order of steps or order for performing certain actions is immaterial, as long as the methods described herein remains operable. Moreover, two or more steps or actions can be conducted simultaneously.

As used herein, the term "about" modifying the quantity of an ingredient, parameter, calculation, or measurement in the compositions employed in the combination, the cell or the methods of the disclosure refers to the variation in the numerical quantity that can occur. Such variation can be within an order of magnitude, typically within 10%, more typically still within 5%, of a given value or range. Whether or not modified by the term "about," the paragraphs below include equivalents to the quantities. Reference to "about" a value or parameter herein includes (and describes) embodiments that are directed to that value or parameter per se. For example, description referring to "about X" includes description of "X." Numeric ranges are inclusive of the numbers defining the range.

The term "nucleic acid" has its art-established meaning and includes DNA and RNA. Preferred is DNA. In some embodiments, said DNA is circular (also referred to as "plasmid").

The term "inhibitory RNA", as used herein, refers to RNA molecules that inhibit or reduce a nucleic acid transcription or translation in a sequence-specific manner. In some embodiments, the inhibitory RNA is an RNA molecule that inhibit or reduce the expression of a gene. In some embodiments, the inhibitory RNA is selected from the group consisting of a shRNA, a miRNA and a siRNA.

The "inhibitory RNA" may refer to a single, double, or tripartite RNA molecule (e.g., an siRNA, an shRNA, an miRNA, a piRNA, etc.) that exerts an effect on a biological process by interacting with one or more components of the RNAi pathway including but not limited to Drosha, RISC, Dicer, etc. The RNA interference process includes, but is not limited to, gene silencing by degrading mRNA, attenuating translation, interactions with tRNA, rRNA, hnRNA, cDNA and genomic DNA, inhibition of as well as methylation of DNA with ancillary proteins. Cognate binding sites may be located in the 3'-untranslated region (3'-UTR), the 5'-UTR or in the coding sequence (CDS). In some embodiments, the binding site is in the 3'-UTR.

The term "siRNA" or "short interfering RNA", as used herein, refers to a double-stranded RNA molecule that comprises typically 20-24 base pairs in length, and operates within the RNA interference pathway. Further information about design, structure and function of siRNAs can be found, e.g., in Elbashir S.M., et al., Nature 411:494-498 (2001).

The term "shRNA" or "short hairpin RNA" or "small hairpin RNA", as used herein, refers to an RNA molecule that forms a hairpin loop that target mRNA for transcript cleavage and/or translational attenuation. Further information about design, structure and function of shRNAs can be found, e.g., in Paddison P.J., et al., Genes & Dev. (2002) 16: 948-958 and Moore et al. Methods Mol Biol. 2010; 629: 141-158.

The term "miRNA" or "microRNA" or "miR", as used herein, refers to small, single stranded, non-coding RNA molecules. Without wishing to be bound by a specific theory, miRNA exploits the RNA Induced Silencing Complex (RISC) which utilizes the seed region (positions 2-7) of the miRNA guide strand to target the mRNA for transcript cleavage and/or translational attenuation. A perfect match to the target mRNA is not required. Further information about design, structure and function of miRNAs can be found, e.g., in Riolo et al., Methods Protoc. 4, 1-20 (2021) and Saliminejad et al., J Cell Physiol. 2019 May;234(5):5451-5465.

The term "gene", as used herein, is defined to include both transcribed and non-transcribed elements. Thus, for instance, a gene can include any non-transcribed enhancer and/or promoter (i.e., genomic DNA) that plays a role in determining the level, timing, or tissue specificity of expression of a particular mRNA transcript or non-coding RNA. In addition, the 5' UTR, ORF, 3' UTR and introns are included as elements of a gene.

The terms "silencing", "inhibition" or "reduction" of a nucleic acid transcription or translation are used interchangeably and are defined as a reduction in gene transcription or translation by an inhibitory RNA that can be measured by any number of methods including PCR-based methods, Northern blot analysis, Branched DNA, western blot analysis, and other art recognized techniques.

To the extent the first nucleic acid is an inhibitory RNA (as opposed to encoding it), said RNA may be modified, for example to increase its stability in a biological, such as cellular environment or to increase specificity. For example, specificity modifications can be incorporated into any inhibitory RNA in order to decrease off-targeting. Such specificity modifications can be an aspect of on-targeting. Further descriptions of modifications that enhance stability and/or specificity include those described in WO 2005/097992, WO 2007/095387, WO 2008/036825, WO 2008/147837, WO 2009/012173 and U.S. 10/551,350, 11/619,993, and 11/857,732, the disclosures of which are incorporated by reference. Modifications to the internucleotide linkages that can enhance overall stability or enzymatic processing can include phosphorothioates, phosphorodithioates, alkylphosphonates, phosphonoacetates, phosphonoacetamides, phosphonoacetic acid esters, phosphonamidates, phosphonoalcohols, phosphonoalcohol esters, phosphonoformates, boranophosphonates, peptide nucleic acids, and more. Similarly, chemically modified nucleotides having modifications to the sugar structures can be included to enhance or alter oligonucleotide stability, functionality, enzymatic processing, and specificity. Possible modifications to the sugar ring structure include 2'-O-alkylribose, 2'-O-methyl, 2'-fluoro, 2'-halo-2'-deoxyribose, 2'-deoxyribose, 2' amino-2'-deoxyribose, 2'-thio-2'-deoxyribose, arabinose, L-ribose, 2'-halo-2'-deoxyarabinose, 2'-O-alkylarabinose, 2'-amino-2'-deoxyarabinose, 2'-thio-2'-deoxyarabinose, 2'-O, 4'-C-methylene bicycloribose ("locked nucleic acid"), 4'-aminoalkylribose, 5'-aminoalkylribose, 4-thioribose, and more.

Even though being referred to as "RNA" herein, inhibitory RNAs in accordance with the disclosure may comprise one or more deoxyribose moieties, for example, but not limited to cases where the inhibitory RNA is implemented as an antisense RNA.

Said inhibitory RNA (the first nucleic acid or encoded by said first nucleic acid) recognizes a binding site in said third nucleic acid. Upon binding to said binding site, said inhibitory RNA exerts control on what is to be transcribed or translated from said third nucleic acid, e.g., a gene of interest. In particular, said inhibitory RNA represses or reduces transcription and or translation of said third nucleic acid.

"Viral particles", as used herein, are particles comprising nucleic acid associated with one or more viral proteins and/or packaged into a capsid formed by one or more viral proteins. Viral particles may furthermore comprise an envelope which is a lipid membrane, typically equipped with one or more viral proteins. Adeno-associated viral (AAV) particles do not comprise such an envelope, while retroviral, including lentiviral particles do. The lipid membrane of enveloped viral particles may be inherited from the cell producing said particles. A cell line capable of producing viral particles ("production cell" or "production cell line") is subject of a further aspect of the present disclosure; see below. The terms "viral particles" and "viral vectors" are used equivalently herein. In some embodiments, said retroviral particles are lentiviral particles.

The combination of nucleic acids of the first aspect is useful for making viral particles, more specifically for transfecting a cell, wherein said transfecting renders said cell capable of producing said viral particles. While the transfected cell (see further below) contains all components of the combination, the produced viral particles will generally not do so, in particular, they will generally not contain said first and said at least one second nucleic acids.

Viral particles in accordance with the disclosure, while sharing features with their naturally occurring counterparts (viruses), preferably contain heterologous nucleic acids (which, in the course of said production, will be incorporated into said particles). Viral particles in accordance with the disclosure are capable of infecting cells, for example mammalian cells, including human cells, which renders them suitable as vectors for transferring nucleic acids into said cells.

The present disclosure refers to different types of cells. First, there is a cell which is to be transfected with the combination of the first aspect. Such cell may be an established cell line or off-the-shelf cell line. Such cell lines are exemplified further below and include HEK293 cells, CAP^{®} (immortalized human amniocyte cell) cells etc. Secondly, upon transfection of the cell to be transfected, a cell in accordance with the fourth aspect is obtained. This cell is also referred to as "production cell" because it is equipped with the components of the combination of the first aspect and therefore capable of producing viral particles. Thirdly, and upon harvesting the viral particles produced by the production cell, the user may choose a target cell which is to be brought into contact with said viral particles. Said bringing into contact will deliver the nucleic acid of interest in accordance with the first aspect and embodiments thereof to said target cell which in turn becomes genetically modified by said nucleic acid of interest.

Said harvesting is generally performed about 12 to about 96 hours, about 24 to about 72 hours, such as for example, about 48 hours after transfection. In a further embodiment, said cells continuously produce said viral particles.

Such gene of interest, while subject of a preferred embodiment, is not a requirement of the combination of the first aspect. Rather, said first aspect provides a tool which enables the user to (i) equip said third nucleic acid with a gene of interest (be it by cloning or gene synthesis), thereby obtaining a final combination of nucleic acids to be transfected in a production cell, (ii) transfect said cell, wherein said cell will benefit from interaction of said inhibitory RNA with said binding site, (iii) harvest the viral particles obtained from said cell, and (iv) use them for the intended purpose which involves transfer of the heterologous nucleic acid ("nucleic acid of interest") into a given target cell.

In view of the option of gene synthesis, said third nucleic acid may comprise but does not have to, one or more cloning sites for said nucleic acid of interest.

The terms "helper nucleic acid" and "helper protein" (sometimes collectively referred to as "helper functions") include those nucleic acids and proteins which are generally referred to in the art as providing helper functions. In this, stricter, sense, these terms are derived from the notion of a helper virus, the helper virus being Adenovirus, herpes viruses, such as HSV-1, HSV-2, HCMV, or derived from other viruses capable of providing helper functions needed for viral replication and production. In one embodiment of this invention, the helper virus is Adenovirus in case of adeno-associated viral particles.

Herein the terms "helper nucleic acid(s)", "helper protein(s)" and "helper function(s)" are used in a wider sense and refer, in case of AAV, also to the AAV nucleic acids which are the Rep and Cap genes, which in turn encode elements of the replication machinery and the viral capsid, respectively (also known as "packaging functions"). Helper nucleic acids and helper protein in accordance with the disclosure provide all functions necessary for obtaining said viral particles. In particular, they serve to replicate and package the third nucleic acid. Helper functions are often provided in trans, but do not have to be (see further below for preferred placements of the nucleic acids of the combination of the first aspect on plasmids).

Of note, at least one of said helper functions may be provided by the cell which is to be transfected with the combination of the first aspect. As a consequence, it is not required that the combination of the first aspect, in particular said at least one second nucleic acid(s) in accordance with the first aspect provide all necessary helper functions. What matters is that the sum of helper functions provided by said combination plus the helper functions provided by said cell provide all necessary helper functions. The set of necessary helper functions may be distributed between said combination and said cell in all conceivable ways, wherein generally at least one of said helper functions is provided by said combination.

Having said that, the present disclosure also provides a combination comprising or consisting of (a) a first nucleic acid encoding or being an inhibitory RNA; and (c) a third nucleic acid comprising a binding site for said inhibitory RNA. In some embodiments, such combination is employed for transfecting a cell to be transfected which on its own provides all necessary helper functions.

On the other hand, also in those cases where it is intended to have the cell provide all necessary helper functions, making use of the combination of the first aspect is envisaged. To explain further, in such a case said at least one second nucleic would be used for the purpose of generating, in a first step, a cell which subsequently will be equipped with all necessary helper functions. In a second step, the cell would be transfected with said first and said third nucleic acid or any embodiments thereof. Said two steps can be carried out in any order or concomitantly. In some embodiments, said first step precedes said second step. In other embodiments, the transfection in accordance with the first step is stable transfection. Alternatively, the second step is transient transfection. Once both steps are completed, a cell in accordance with the fourth aspect is obtained.

In a variation of the above, a cell line to be transfected may be used, e.g., off-the-shelf or an established cell line which provides a subset of the necessary helper functions. In that case, the equivalent of said second step mentioned above would be transfecting with a combination of the first aspect, wherein said at least one helper nucleic acid as comprised in said combination serves to complete the set of helper functions, such that after transfecting said off-the-shelf or established cell line with said combination, the obtained cell line (a cell line in accordance with the fourth aspect) is equipped with the necessary set of helper functions.

For AAV particles, preferred Adenovirus-derived helper nucleic acids or helper proteins include E2A, E4 and VA. They mediate replication. VA acts as a helper nucleic acid, more specifically an RNA (see, e.g., Maier et al., Viruses 12(6): 662 (2020)), while E2A and E4 are translated into helper proteins. Of note, helper functions do not have to originate from Adenoviridae, but may also originate from Herpesviridae. Given that helper functions mediate replication, the skilled person can determine without further ado in a promoter binding assay or promoter activation assay whether a given polypeptide encoded by a gene of a member of Adenoviridae or Herpesviridae is capable of binding and activating an AAV promoter. In some embodiments, the above-mentioned helper functions originate from Adenovirus type 5.

A further helper protein is encoded by the E1 region of Adenovirus. E1 proteins may be provided by a suitable established cell line, such as HEK293 cells or CAP^{®} cells (Schiedner G, et al., Hum Gene Ther. 11, 2105-2116 (2000)) but may also be provided as helper protein (or encoded by a helper nucleic acid) which are components of the combination of nucleic acids of the first aspect of the disclosure.

Of note, Rep and Cap (packaging functions and helper functions in accordance with the present disclosure) may also be provided by a suitable cell line such HeLaS3 (Martin J., et al. Human Gene Therapy Methods 24,1-17 (2013)).

In some embodiments, a sufficient set of helper functions for the production of AAV particles comprises or consists of Rep, Cap, E2A, E4, VA, and E1.

In some embodiments, the set of helper proteins for the production of AAV particles is the set comprising or consisting of Adenovirus proteins E1a, E1b-55K, E2a and E4orf6; see Samulski & Shenk, J. Virol. 62, 206-210 (1988). This set of helper proteins is to be used together with the helper nucleic acid VA (see above). Another preferred set of helper proteins is the set comprising or consisting of HSV-1 proteins UL5, UL8, UL52 and UL29, the former three forming the helicase/primase complex and the latter encoding the single-stranded DNA-binding protein; see also Weindler & Heilbronn, J. Virol. 65, 2476-2483 (1991). In order to obtain a sufficient set of helper functions based on the two sets disclosed above, Rep and Cap have to added.

The way how such helper functions are provided is not particularly limited. In some embodiments, said helper functions are provided by helper viruses, such as herpes viruses or adenoviruses (see, e.g., Toublanc et al., J. Gene Med. 6, 555-564 (2004). In an embodiment, said helper viruses are replication defective. In an embodiment, said helper viruses are infective.

Further alternatives to said helper proteins and, where applicable, helper nucleic acids, which alternatives meet the functional requirement as laid down above can be determined by the skilled person without further ado. For example, a protein coding sequence under the control of a promoter may be combined with one or more helper functions and production of viral particles used as read-out in order to determine a useful set of helper functions. Further information about helper functions can be found, for example, in McPherson et al., Virology 147, 217-222 (1985).

To the extent the present disclosure relates to retroviral particles, preferably, lentiviral particles, in some embodiments, helper nucleic acids/helper proteins include gag, pol, env, rev and optionally tat. In some embodiments, the helper function of env is a gene encoding a glycoprotein of vesicular stomatitis virus (VSV-G) owing to its broad tropism (host range); see, for example, Bischof et al., Methods Mol. Biol. 614, 53-68 (2010). The use of heterologous env proteins is also known as pseudo-typing. Pseudo-typing is established in the art and described in, e.g., Steffen, I., & Simmons, G. Current gene therapy, 16(1), 47-55 (2016).

In some embodiments, a sufficient set of helper functions for retroviral particles, in particular lentiviral particles, is the set comprising or consisting of gag, pol, env and rev.

It is understood that instead of these specific helper proteins and helper nucleic acids, functional homologues may be used. Functional homologues exhibit at least 80%, at least 90%, at least 95%, at least 98%, at least 99% sequence identity to the respective parent protein or nucleic acid (preferred parent proteins and nucleic acids being disclosed above), respectively. Said homologues retain their helper function, i.e., the capability to help or trigger replication of the third nucleic acid and packaging it into viral particles. In some embodiments, said capability to trigger replication and packaging is at least 10%, at least 20%, at least 30%, at least 40%, at least 50%, at least 60%, at least 70%, at least 80% or at least 90% of the capability of the parent helper protein or helper nucleic acid.

The present disclosure provides means and methods to control transcription and/or translation of a gene of interest and, to the extent said gene of interest encodes a protein of interest, of said protein of interest. Such control is reduction or inhibition. In some embodiments, this is desirable since the purpose of a cell transfected with the combination of the disclosure is the production of viral particles and generally not the concomitant transcription or translation of a gene of interest. The combination of the disclosure provides for the cell to produce said viral particles while avoiding consumption of cellular resources for any concomitant transcription or translation of the gene of interest.

In addition, certain nucleic acids of interest or genes of interest are or encode products which are toxic for said cell (i.e., for the production cell line). Non-limiting examples of such toxic products include proteins selected from the group consisting of pro-apoptotic proteins, such as RIPK3, proteins interacting with a component of the NPκB pathway, such as TRIF interacting with TLR3, proteins that bind to the nuclear membrane and interfere with rAAV production, such as eGFP-KASH fusion protein, etc. Reducing or inhibiting expression of a toxic gene product is not only a means to secure survival of the production cell, but, as stated above, also to increase production of said viral particles as compared to a setup where both said inhibitory RNA and said binding site are absent.

In some embodiments, said increase in production of viral particles may be at least 1.5-fold, at least 2-fold or at least 5-fold compared to the absence of said inhibitory RNA, preferably compared to the absence of both said inhibitory RNA and said binding site. The examples below provide evidence of a more than 5-fold increase.

The present disclosure is advantageous over art-established systems for inhibiting expression, such as the Tet system or the tamoxifen inducible Cre system. The Tet system requires the use of antibiotics and the presence of an expressed Tet repressor protein binding to TetO sequences. Therefore, the Tet system adds complexity to a system to produce viral particles, and may deliver a slow or delayed inhibitory response. While using tamoxifen (an estrogen receptor modulator) instead of an antibiotic for expression control, disadvantages of the Tet system generally apply as well. The present disclosure overcomes these deficiencies. Of note, and despite dispensing with said art-established systems, the present disclosure delivers at least a comparable performance.

Therefore, the present disclosure does not have to rely on any further or alternative means to control transcription or translation of a transgene ("nucleic acid or gene of interest").

In another embodiment, the combination of the disclosure may further comprise the Tet system, the tamoxifen inducible Cre system and/or other means to control transcription or translation of a gene of interest.

In addition, and in terms of advantageous properties, the present disclosure, in particular the generation of a production cell line for producing said viral particles, can be conducted in a "single pot" by providing the first, second and third nucleic acids to the production cells e.g. by simple transfection. In other words, the preferred taxonomic categories of viruses (AAV and retroviruses including lentiviruses) do not require any multiple passaging which is preferred or required for other viral vectors like adenovirus vectors and MVA vectors. Because there are no multiple passages involved, there is no need to further remove proteins like the Tet repressor protein from the final viral vector preparation.

In another embodiment, said inhibitory RNA is a small inhibitory RNA. Non-limiting examples of small inhibitory RNAs are an shRNA, a miRNA, an siRNA or an antisense RNA.

In a further embodiment, more than one inhibitory RNA is used, such as two, three, four, five of more inhibitory RNAs. Each member of such plurality of inhibitory RNAs may, but does not have to, recognize different binding sites. Without wishing to be bound a specific theory, it is considered that the use of more than one inhibitory RNA causes synergistic effects in terms of inhibiting expression of the nucleic acid of interest comprised in the third nucleic acid. When making use of more than one inhibitory RNA, the molecular architecture is not particularly limited. One may use, e.g., two shRNAs, two miRNAs, two siRNAs, two antisense RNAs, one shRNA and one miRNA, one shRNA and one siRNA etc.

In some embodiments, the third nucleic acid comprises a nucleic acid of interest.

In some embodiments, said nucleic acid of interest comprises a gene to be transcribed, in particular a gene to be transcribed in a production cell of the present disclosure. Such gene would be transcribed into a transcript which encodes a protein, or into a non-coding nucleic acid, such as a non-coding RNA. In either case, in some embodiments, the transcript of said gene of interest comprises the region coding for said protein or comprises said non-coding RNA, and furthermore comprises said binding site in accordance with the combination of the first aspect.

In other embodiments, said viral particles that are produced by using the combination of the disclosure are adeno/associated viral (AAV) particles and said third nucleic acid comprises inverted terminal repeats (ITRs). Alternatively, said viral particles that are produced by using the combination of the disclosure are retroviral particles, such as lentiviral particles, and said third nucleic acid comprises long terminal repeats (LTRs).

In other embodiments, said nucleic acid of interest is or comprises a gene of interest. In some embodiments, said nucleic acid of interest encodes a protein of interest. Said nucleic acid of interest may be coding or non-coding. Yet, a particular strength of the combination of nucleic acids of the present disclosure is to prevent or reduce the production of the protein encoded by said third nucleic acid while, viral particles are being produced by the cell line of the disclosure (when transfected with the components of the combination of nucleic acids of the first aspect). Without wishing to be bound by any particular theory, the inhibitory or reduction action of said inhibitory RNA present in the combination of the first aspect provides for better performance of the production cell line in that the yield of viral particles is substantially increased when compared to an analogous setup where the inhibitory RNA is not present.

In another embodiment, said nucleic acid of interest is transcribed into a non-coding RNA. Such non-coding RNA, when expressed, may be toxic to cells. Expression is inhibited by the inhibitory RNA.

In some embodiments, said inhibitory RNA does not bind to the second nucleic acid(s). This can be measured by mRNA sequencing methods. In other words, said inhibitory RNA is tailored to bind to a site within said third nucleic acid, be it a binding site within said third nucleic acid or a binding site within said nucleic acid of interest to be placed within said third nucleic acid. In structural terms, said inhibitory RNA has less than 100%, less than 98%, less than 95%, less than 90%, less than 80% complementarity or no statistically significant complementarity to any of said second nucleic acid(s).

In one embodiment, said inhibitory RNA has less than 100%, less than 98%, less than 95%, less than 90%, less than 80% complementarity or no statistically significant complementarity to a sequence of a gene endogenous to the cell of the fourth aspect, in order not to inhibit or reduce said gene expression substantially. This can be measured by mRNA sequencing methods. Said endogenous gene is to be held distinct from any nucleic acid of the combination of the first aspect. In other words, an endogenous gene is a gene comprised in a cell line (such as an off-the-shelf or established cell line) which is there prior to transfecting said cell line with a combination of the first aspect. Of note, such endogenous genes may encode functions vital to the cell the inhibition of which is generally undesirable.

In some embodiments, said inhibitory RNA has a lower degree of complementarity or no statistically significant complementarity to any of said second nucleic acid(s). In other embodiments, said inhibitory RNA has a lower degree of complementarity or no statistically significant complementarity to a sequence of a gene endogenous to the cell line of the fourth aspect.In another embodiment of the combination of the first aspect:
(i) said first nucleic acid and said at least one second nucleic acid are comprised in a first plasmid and said third nucleic acid is comprised in a second plasmid;
(ii) said first nucleic acid and said third nucleic acid are comprised in a first plasmid and said at least one second nucleic acid is comprised in at least one second plasmid;
(iii) said first nucleic acid is comprised in a first plasmid, said at least one second nucleic acid is comprised in at least one second plasmid and said third nucleic acid is comprised in a third plasmid;
(iv) said first nucleic acid, said at least one second nucleic acid and said third nucleic acid are comprised in the same plasmid; or
(v) said first nucleic acid is comprised in a first plasmid, and said at least one second nucleic acid and said third nucleic acid are comprised in a second plasmid.

Plasmids carrying second nucleic acids are commonly referred to as "helper plasmids". Similar to the terms "helper function", "helper nucleic acid" and "helper protein" being defined as above, the notion of a "helper plasmid" has the same meaning. In particular, it includes what is sometimes also referred to as "packaging plasmids". In the case of AAV, a plasmid carrying Rep and Cap genes would be called "packaging plasmid" and, in accordance with the disclosure, it would be referred to as a "helper plasmid". Of note, and as known in the art, packaging functions are generally specific for each viral serotype.

As noted above, the set of helper functions provided by said one or more helper plasmids does not have to be comprehensive in the sense that all necessary helper functions are provided, in particular in those cases where a complementing set of helper functions is provided by the chosen type of cell to be transfected.

The number of plasmids may vary and may be adjusted by a person skilled in the art. Typically, but not exclusively, for the production of AAV particles, the at least one second nucleic acid is or are located on one helper plasmid. In the case of retroviruses, in particular lentiviruses, and depending on the generation (such as second and third generation) of Lentivirus, said second nucleic acids may be located on three or four plasmids. In some embodiments, a first helper plasmid carries the rev gene, for example from HIV1. In another embodiment, a second helper plasmid carries the gag and pol genes, for example from HIV1. In a further embodiment, a third helper plasmid carries an env gene, for example VSV-G. Helper plasmids and helper functions are well known in the art and have been described, for example, in Maier A.F., et al. Viruses 2020, 12, 662 and Bulcha J.T., et al. Signal Transduction and Targeted Therapy (2021) 6:53.

In a further embodiment, said first nucleic acid encodes said inhibitory RNA and comprises a promoter selected from the group consisting of (i) an RNA polymerase III promoter; (ii) a small RNA-expressing promoter, such as a U6 promoter or H1 promoter; (iii) an RNA polymerase II promoter; and (iv) a promoter with activity in eukaryotic cells, such as CMV promoter. Those skilled in the art can determine the appropriate promoter to be used for the first nucleic acid.

It is understood that said the plasmid/s comprising the at least second nucleic acid comprise the necessary elements for transcription of said helper nucleic acids, or those which are necessary to complement those helper functions which are provided by cell line to be transfected (such as off-the-shelf or established cell lines). Furthermore, the plasmid comprising the third nucleic acid and said binding site for the inhibitory RNA and, where applicable, said nucleic acid of interest, contains such necessary elements as well. This ensures that, in the absence of said inhibitory RNA (e.g., in a target cell to be transfected with said viral particles), transcription, and, where applicable, translation of said nucleic acid of interest or of said protein of interest ensues. Optionally, the corresponding nucleic acids as recited in the first aspect may already comprise these elements. Said necessary elements include promoters. In addition, enhancers may be present as well.

In one embodiment, said inhibitory RNA, when transcribed, inhibits the transcription of said nucleic acid of interest and/or the expression of said protein of interest.

In a further embodiment, said binding site is located in the nucleic acid of interest, for example, in an untranslated region (UTR) of said nucleic acid of interest, such as the 3'-UTR or the 5'-UTR.

In one embodiment, said binding site is a sub-sequence of said nucleic acid of interest. Small inhibitory RNAs, such as shRNAs, miRNAs, siRNAs and antisense RNAs can easily be tailored to bind a nucleic acid of interest. Generally speaking, a certain degree of complementarity of said inhibitory RNA to said nucleic acid of interest or, more generally, to said third nucleic acid is preferred. Complementarity entails the capability of the inhibitory RNA to hybridize or, in other words, perform Watson-Crick base pairing with said third nucleic acid. In some embodiments, complementarity is over at least 80%, at least 90%, at least 95%, at least 98% or 100% of the length of said inhibitory RNA. In other embodiments, for small inhibitory RNAs, there are 3 or less, 2 or less, one, or no mismatch between the small inhibitory RNA and the third nucleic acid. Without wishing to be limited by a specific theory, it is considered that complementarity requirements are lower for miRNAs than for shRNAs, siRNAs and antisense RNAs. In some embodiments, the small inhibitory RNAs, such as shRNAs, miRNAs, siRNAs and antisense RNAs, comprise higher degrees of complementarity including 100% complementarity. In some embodiments, miRNAs, depending on their mechanism of action, may exhibit less than 100% complementarity to the nucleic acid of interest or to the third nucleic acid of interest. Whether a certain level of complementarity which is below 100% is sufficient, can be determined, for example, by measuring the degree of inhibition of transcription of said nucleic acid of interest, e.g., by mRNA sequencing and/or by quantifying the amount or number of viral particles produced.

In an alternative embodiment, said binding site is unrelated to said nucleic acid of interest. Such unrelatedness confers distinct advantages in that it does not entail a requirement of said combination to be tailored to a particular nucleic acid of interest. In other embodiments, when said binding site is unrelated to said nucleic acid of interest, use is made of a generic binding site which is incorporated into said third nucleic acid (irrespective of the presence of a nucleic acid of interest in said third nucleic acid). This approach allows for a universal first nucleic acid (and universal plasmid comprising said first nucleic acid) that comprises the inhibitory RNA and which does not require adaptation, neither to the virus family or viral serotype, nor to the specific nucleic acid of interest.

In one embodiment, said viral particles produced by the combination of the first aspect are AAV particles and said helper nucleic acids and helper proteins comprise Rep, Cap, E2A, E4, VA, and E1. In other embodiments, said viral particles are retroviral particles, such as lentiviral particles and said helper nucleic acids and helper proteins comprise gag, pol, env, and rev. In some embodiments, certain helper functions may be provided by the cell to be transfected with the combination of the first aspect. In such a case, and as stated above, the set of helper functions in accordance with item (b) of the first aspect of the disclosure need not be sufficient for obtaining said viral particles, as long as the cell provides any missing helper functions which make the complete set of helper functions (across the combination of the disclosure and the cell) sufficient for the production of viral particles.

In some embodiments, said combination of the first aspect does not comprise further means suitable for inhibiting transcription or translation of a gene of interest. In some embodiments, the combination does not comprise components of inducible gene expression systems, such as the Tet system and/or the Tamoxifen inducible system. Inducible gene expression systems are well known in the art and have been described, for example, in Kallunki T., et al. Cells 2019, 8, 796.

Just for the sake of completeness, the following description of an exemplary Tet system (also known as "T-Rex") is given. In the T-REx system, the gene of interest is flanked by an upstream CMV promoter and two copies of tetracycline operator 2 (TetO2) sites. Expression of the gene of interest is repressed by the high affinity binding of TetR homodimers to each TetO2 sequences in the absence of tetracycline. Introduction of tetracycline results in binding of one tetracycline on each TetR homodimer followed by release of the TetR homodimer from the TetO2. Unbinding of TetR homodimers form the TetO2 results in de-repression of the gene of interest. See, for example, Hillen W. and Berens. Annu Rev Microbiol. 1994;48:345-69; W Hillen et al. J Mol Biol. 1983 Sep 25;169(3):707-21; Kathleen Postle et al. Nucleic Acids Research, Volume 12, Issue 12, 25 June 1984, Pages 4849-4863; and Feng Yao et al. Human Gene Therapy. Sep 1998.1939-1950.

In one embodiment, said inhibitory RNA is an shRNA and comprises the sequence of SEQ ID NO: 1 or a sequence at least 80%, at least 85%, at least 90%, at least 95%, at least 98%, at least 99%, at least 99.5%, or 100% identical to the sequence of SEQ ID NO: 1. In some embodiments, the binding site of said shRNA of SEQ ID NO: 1 or comprising a sequence at least 80%, 85%, 90%, 95%, 98%, 99%, or 99.5% identical to the sequence of SEQ ID NO: 1 comprises the sequence of SEQ ID NO: 2 or a sequence at least 80%, at least 85%, at least 90%, at least 95%, at least 98%, at least 99%, at least 99.5%, or 100% identical to the sequence of SEQ ID NO: 2.

In another embodiment, said inhibitory RNA is an shRNA and comprises the sequence of SEQ ID NO: 3 or a sequence at least 80%, at least 85%, at least 90%, at least 95%, at least 98%, at least 99%, at least 99.5%, or 100% identical to the sequence of SEQ ID NO: 3. In some embodiments, the binding site of said shRNA of SEQ ID NO: 3 or comprising a sequence at least 80%, at least 85%, at least 90%, at least 95%, at least 98%, at least 99%, at least 99.5%, or 100% identical to the sequence of SEQ ID NO: 3 comprises the sequence of SEQ ID NO: 4 or a sequence at least 80%, at least 85%, at least 90%, at least 95%, at least 98%, at least 99%, at least 99.5%, or 100% identical to the sequence of SEQ ID NO: 4.

As shown in the Examples, this particular shRNA targets a binding site within the coding sequence of the gene of interest. As such, it is demonstrated that inhibitory RNAs may be used which are specific for the gene of interest on the one hand, and RNAs which are not specific for the gene of interest but bind to a target site in a non-coding region of said gene of interest.

In another embodiment, said inhibitory RNA is a miRNA and comprises the sequence of SEQ ID NO: 5 or a sequence at least 80%, at least 85%, at least 90%, at least 95%, at least 98%, at least 99%, at least 99.5%, or 100% identical to the sequence of SEQ ID NO: 5. In some embodiments, the binding site of said miRNA of SEQ ID NO: 5 or comprising a sequence at least 80%, 85%, 90%, 95%, 98%, 99%, or 99.5% identical to the sequence of SEQ ID NO: 5 comprises the sequence of SEQ ID NO: 6 or a sequence at least 80%, at least 85%, at least 90%, at least 95%, at least 98%, at least 99%, at least 99.5%, or 100% identical to the sequence of SEQ ID NO: 6.

As common in the design of inhibitory RNAs, the inhibitory RNA comprises a strand which comprises a sequence which is at least 80%, at least 85%, at least 90%, at least 95%, at least 98%, at least 99%, at least 99.5%, or 100% identical to a sequence comprised in the respective binding site; see the highlighted sequences in the Examples for illustration.

In a second aspect, the present disclosure relates to the use of the combination of the first aspect or any of its embodiments for transfection of a cell.

In some embodiments, said use is in vitro or ex vivo and/or said cell is a cell in culture.

The embodiments defined above of the combination of the first aspect are also defined for the use of the second aspect.

In a third aspect, the present disclosure provides a method of transfecting a cell, said method comprising bringing a cell into contact with the components of the combination of the first aspect or any of its defined embodiments.

The embodiments of the below fourth aspect of the disclosure (a cell) are also applicable to the third aspect.

In some embodiments, said transfecting is (a) transient with regard to said first, said at least one second and said third nucleic acid, or (b) stable with regard to said first nucleic acid and/or at least one of the at least one second nucleic acids (being the first and/or second nucleic acid integrated into a host cell genome), and transient as regards the remainder of the nucleic acids.

Alternatively, the transfecting is (c) stable with regards to the third nucleic acid (including or not the nucleic acid of interest), for example, the third nucleic acid is integrated in a host cell genome. HeLaS3 cells are an example in that respect. In some embodiments, the stably integrated third nucleic acid comprises the binding said for the inhibitory RNA.

In that case, transfecting may be, but does not have to be, transient as regards the remainder of the nucleic acids. In some embodiments, the stable transfection precedes the transient transfection.

Said transfection can be carried out using one of a variety of methods known to those skilled in the art. For example, transfection reagents, such as polyethyleneimine (PEI) or polybrene may be used. Electroporation is another established transfection method.

In some embodiments, said method is performed in vitro or ex vivo.

In a fourth aspect, the present disclosure provides (a) a cell obtained by the method of the third aspect or any of its defined embodiments; and/or (b) a cell comprising the components of the combination of the first aspect or any of the defined embodiments of the first aspect.

The term "cell", "cell line" and "cell in culture" are used equivalently herein.

In some embodiments, said cell line is not the human body, at the various stages of its formation and development.

In one embodiment of said cell line, the first nucleic acid and/or at least one of the at least one second nucleic acids is/are integrated into the genome of said cell. In another embodiment, the third nucleic acid is integrated into the genome of said cell. See, e.g., Escandell et al., Biotechnol. Bioeng. 120, 2578-2587 (2023). In another embodiment, the third nucleic acid comprising a nucleic acid or a gene of interest is integrated into the genome of said cell.

As mentioned above, helper functions may be provided by helper viruses. This applies in particular, but not only, to the above embodiments where the cell line is stably transfected with said third nucleic acid, i.e., said cell line has said third nucleic acid integrated into its genome.

In one embodiment, said cell line is of mammalian or insect origin. In some embodiments, said cell line of mammalian origin is of human origin. Non-limiting examples of mammalian cells of human origin are HEK-293, HeLa, HeLaS3 and CAP^{®}. In other embodiments, said cell line of insect origin is Sf9.

In a fifth aspect, the present disclosure provides a method of producing viral particles carrying a nucleic acid of interest, said method comprising (a) bringing a cell into contact with the components of the combination of the first aspect, more specifically those embodiments of the first aspect which require the presence of a nucleic acid of interest; and (b) allowing formation of said viral particles to occur. In some embodiments, said method is an in vitro or ex vivo method and/or said cell is a cell in culture.

In some embodiments, for the transfection to occur, the cells viability is determined and when the viability is higher than 95%, the cells are ready for the transfection.

In some embodiments, the method of producing viral particles of the fifth aspect of the disclosure comprises a triple transfection with (i) a plasmid comprising helper nucleic acids, such as a helper plasmid and comprising also an inhibitory RNA (ii) a plasmid or a transgene plasmid comprising a nucleic acid of interest or gene of interest (that may or may not be toxic) and a binding site for said inhibitory RNA; and (iii) a plasmid comprising Rep and Cap, also referred to as a packaging plasmid.

In some embodiments, the molar ratio of the transgene plasmid to packaging plasmid to pHelper plasmid DNA is 1:3:1. Different ratios may be used and can be determined by the skilled person without further ado, including optimization for given plasmids. In some embodiments, after the transfection, the cells are incubated in a 37ºC incubator with a humidified atmosphere of 8% CO2.In some embodiments of the fifth aspect, the cells are harvested, lysed, and clarified or filtered by methods known to those skilled in the art, to obtain the viral particles.

In some embodiments of the method of the fifth aspect, the third nucleic acid of said combination comprises a gene of interest which encodes a protein of interest, and wherein (a) said protein is not produced or is not substantially produced; and/or (b) the production of said viral particles is increased as compared to the absence of said first nucleic acid encoding or being an inhibitory RNA.

In some embodiments, the reduction of production of the protein is at least of 2%, at least of 5%, at least of 7%, at least of 10%, at least of 15% or at least 20%. In some embodiments, the production of said viral particles in increased at least 1.5-fold, at least 2-fold, at least 5-fold or at least 10-fold, compared to a method of producing viral particles in the absence of the first nucleic acid encoding or being an inhibitory RNA.

In some embodiments of said combination, use, method, or cell line of any one of the preceding aspects, said nucleic acid of interest is or encodes a vaccine, is a therapeutic gene or encodes a therapeutic protein, is a diagnostic gene, or encodes a protein capable of binding to a cognate binding partner.

In some embodiments, said therapeutic gene or said therapeutic protein complements a genetic disorder and/or a disorder related to the under-expression of the naturally occurring counterpart of said gene or protein; encodes or is a chimeric antigen receptor (CAR); encodes or is a base editor; or said cognate binding partner is a biomolecule of diagnostic or analytical interest, for example, said biomolecule being a protein.

In a further aspect, the present disclosure relates to a second combination of nucleic acids for the production of viral particles, said combination comprising or consisting of (a) a first nucleic acid encoding or being an inhibitory RNA; (b) at least one second nucleic acid comprising helper nucleic acids necessary for production of said viral particles, and/or encoding helper proteins necessary for said production; and (c) a third nucleic acid comprising a nucleic acid of interest. In some embodiments, said nucleic acid of interest is a gene of interest. In other embodiments, said gene of interest encodes a protein of interest. Of note, said second combination does not comprise the binding site of the combination of the first aspect.

The different embodiments of the combination of the first aspect apply herein *mutatis mutandis.*

As shown in the Examples, it surprisingly turned at that the presence of an inhibitory RNA provides beneficial effects even in the absence of a cognate binding site for said inhibitory RNA. Even though in such a setting, transcription and/or translation of said gene of interest is not inhibited or is not inhibited to a lesser degree, as compared to the presence of a binding site in accordance with the first aspect, production of a protein of interest encoded by said gene of interest is nevertheless increased.

Related to said second combination, the present disclosure provides, in further aspects: a use of said second combination for transfection of a cell; a method of transfecting a cell, said method comprising bringing a cell into contact with the components of said second combination; a cell line obtained by said method and/or comprising the components of said second combination; and a method of producing viral particles carrying a nucleic acid of interest, said method comprising bringing a cell into contact with the components of said second combination and allowing formation of said viral particles to occur.

Preferred embodiments of the second, third, fourth, and fifth aspect apply *mutatis mutandis* to said further aspects of the second combination.

### EXAMPLES

### Example 1: Cloning of plasmids to produce GFP-expressing AAV particles

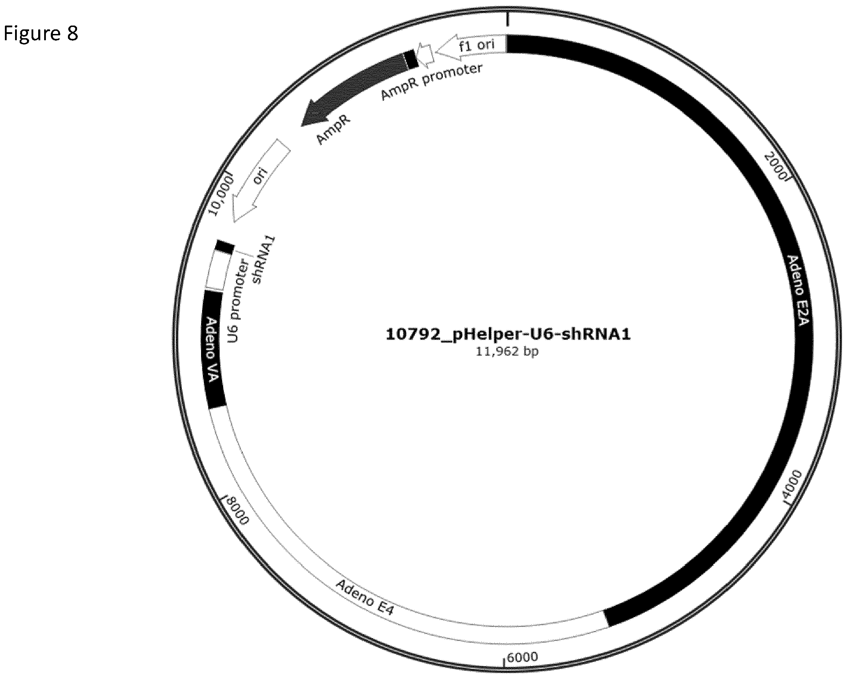

A synthesized DNA fragment consisting of the human U6 promoter and an shRNA (SEQ ID NO: 1) were cloned via Gibson assembly into the Pme1 site upstream of the E4 gene of the helper plasmid pHelper, resulting in plasmid pHelper-U6-shRNA1 (Fig 8).
shRNA (SEQ ID NO: 1):
accggcacagtgctaaccagaaaatcatgagattttctggttagcactgtgcttttttg

The shaded regions of the shRNA are the two reverse complementary arms of the shRNA forming the hairpin.

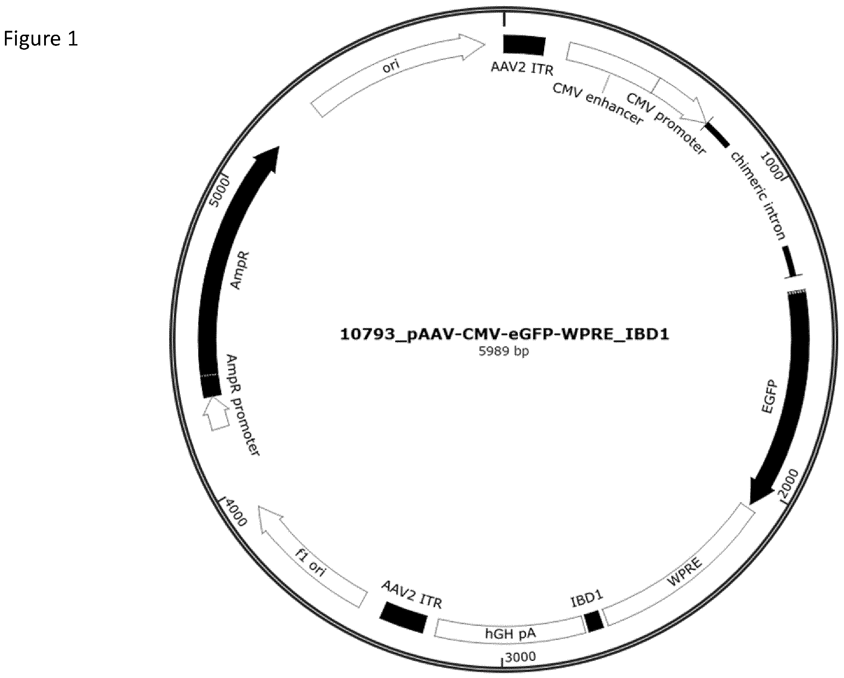

For the generation of the transgene plasmid pAAV-CMV-eGFP-WPRE-IBD1 (Fig. 1), a 47 bp fragment which contains the inhibitor binding domain 1 (SEQ ID NO: 2; IBD1), here the target site of shRNA, was cloned downstream of the WPRE element into the BglII and XhoI sites of plasmid pAAV-CMV-eGFP-WPRE.
IBD1 (SEQ ID NO: 2): ggaagaatgtgcacagtgctaaccagaaaattcaggacaagactagt

The shaded region of IBD1 is the shRNA binding site.

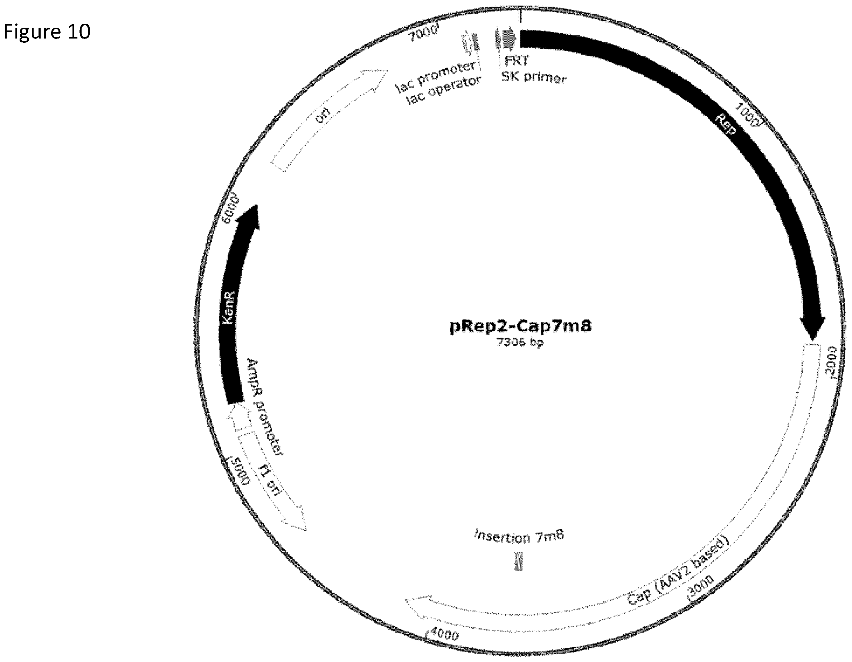

The packaging plasmid used was pRep2-CAP2.7m8, corresponding to the serotype AAV2-7m8 (Fig. 10).

### Example 2: Production of recombinant AAVs expressing GFP

The production of recombinant AAVs (rAAV) of the serotype AAV2-7m8 was performed in 30 mL scale by triple transfection of Viral Production Cells 2.0 (VPCs 2.0 from Gibco^{™}) with the transgene plasmid pAAV-CMV-eGFP-WPRE-IBD1, the helper plasmid pHelper-U6-shRNA1 and the packaging plasmid pRep2-CAP2.7m8. The transfected combinations are listed in Table 1 below:

**Table 1: Plasmids combination transfected into VPC cells.**

| | **Vector plasmid** | **Helper Plasmid** | **Packaging Plasmid** | **Resulting serotype** |
|---|---|---|---|---|
| 1 | pAAV-CMV-eGFP-WPRE-IBD1 | pHelper-U6-shRNA1 | pRep2-CAP2.7m8 | AAV2.7m8 |
| 2 | pAAV-CMV-eGFP-WPRE | pHelper-U6-shRNA1 | pRep2-CAP2.7m8 | AAV2.7m8 |
| 3 | pAAV-CMV-eGFP-WPRE | pHelper | pRep2-CAP2.7m8 | AAV2.7m8 |

On the day of transfection, the viability was determined using a Via1-cassette and Nucleocounter. The cells reached a density of 4.5 to 6.0 × 10⁶ viable cells/mL and viability of ≥95% to proceed with the transfection. The cells were diluted to a final density of 3 × 10⁶ viable cells/mL with fresh Viral Production Medium supplemented with 4 mM GlutaMAX^{™}. Cells were counted again with a Via1-cassette and Nucleocounter to ensure they were at the target density of 3 × 10⁶ viable cells/mL.

The cells were incubated in a 37°C incubator with a humidified atmosphere of 8% CO2 on an orbital shaker until the DNA/transfection complexes were ready.

The molar ratio of transgene plasmid to packaging plasmid to pHelper plasmid DNA was 1:3:1. A total plasmid DNA of 1.5 µg was used for every mL of culture to be transfected.

The plasmid DNA was diluted with Viral-Plex^{™} Complexation Buffer to a final volume of 10% of the culture volume to be transfected. To a new tube, AAV-MAX Transfection Booster was added at 3 µL per mL of culture to be transfected followed by AAV-MAX Transfection Reagent at 6 µL per mL of culture to be transfected. The pre-mixed AAV-MAX Transfection Booster/ Reagent was slowly added to the diluted plasmid DNA. The complexes were incubated at room temperature for 20 to 30 minutes, then the solution was slowly pipetted into the shaker flask(s) while swirling.

AAV-MAX Enhancer was then added at 10 µL per mL of culture. The flask(s) were gently swirled to mix the cells. The cells were incubated in a 37°C incubator with a humidified atmosphere of 8% CO2 on an orbital shaker.

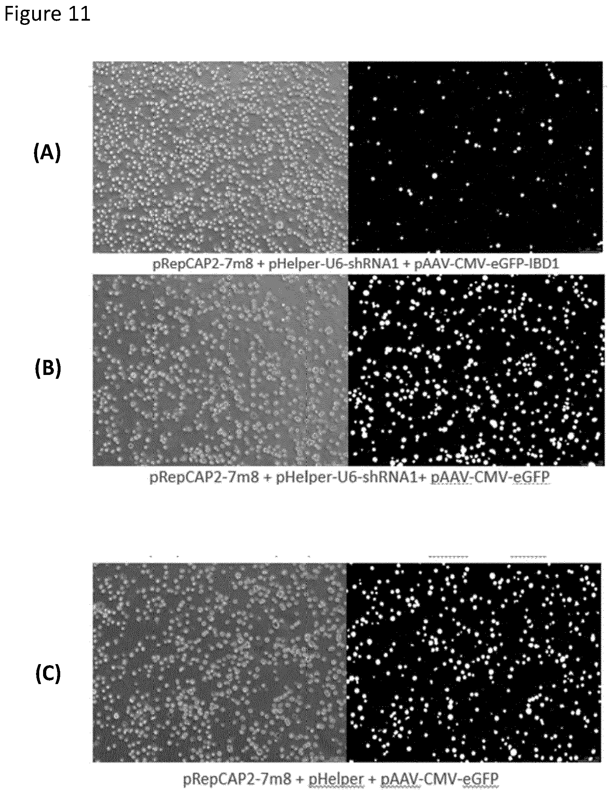

After 72 hours, brightfield and fluorescence pictures were taken from each transfection (Fig. 11). As shown in Fig. 11, the number of green- fluorescent cells, as a direct measure of the expression of the vector encoded transgene, is significantly reduced when shRNA1 is expressed in the presence of the eGFP vector plasmid which contains IBD1 (Fig. 11A), but not when one or both of shRNA1 or IBD1 components are not present.

In a next step, recombinant AAV productions were performed with and without suppression of eGFP expression during production for different AAV serotypes: AAV2, AAV5, AAV6, AAV8 and AAV9 using the following combinations of transgene, helper and packaging plasmids (see Table 2 below). For each condition, rAAV productions were set up in triplicate. Transfections were performed as described above.

**Table 2: Plasmids combinations transfected into VPC cells.**

| | **Vector plasmid** | **Helper Plasmid** | **Packaging Plasmid** | **Resulting serotype** |
|---|---|---|---|---|
| 1 | pAAV-CMV-eGFP-WPRE-IBD1 | pHelper | pRep2-CAP2 | AAV2 |
| 2 | pAAV-CMV-eGFP-WPRE-IBD1 | pHelper-U6-shRNA1 | pRep2-CAP2 | AAV2 |
| 3 | pAAV-CMV-eGFP-WPRE-IBD1 | pHelper | pRep2-CAP5 | AAV5 |
| 4 | pAAV-CMV-eGFP-WPRE-IBD1 | pHelper-U6-shRNA1 | pRep2-CAP5 | AAV5 |
| 5 | pAAV-CMV-eGFP-WPRE-IBD1 | pHelper | pRep2-CAP6 | AAV6 |
| 6 | pAAV-CMV-eGFP-WPRE-IBD1 | pHelper-U6-shRNA1 | pRep2-CAP6 | AAV6 |
| 7 | pAAV-CMV-eGFP-WPRE-IBD1 | pHelper | pRep2-CAP8 | AAV8 |
| 8 | pAAV-CMV-eGFP-WPRE-IBD1 | pHelper-U6-shRNA1 | pRep2-CAP8 | AAV8 |
| 9 | pAAV-CMV-eGFP-WPRE-IBD1 | pHelper | pRep2-CAP9 | AAV9 |
| 10 | pAAV-CMV-eGFP-WPRE-IBD1 | pHelper-U6-shRNA1 | pRep2-CAP9 | AAV9 |

### Example 3: Harvesting and clarification of the rAAV particles expressing GFP

The rAAV particles produced in 30ml were harvested 70 to 72 hours post-transfection by adding AAV-MAX Lysis Buffer and MgCh directly to the culture flask (final concentration of 1X and 2 mM, respectively). The flasks were swirled to evenly distribute the lysis buffer. Per flask, Benzonase^{®} nuclease was added to a final concentration of 90 units/mL.

The flasks were incubated at 37 °C for at least 2 hours on an orbital shaker.

The cell-lysate was clarified using a Sartoclear Dynamics^{®} Lab kit. Sartoclear Dynamics^{®} Lab kits are designed for the rapid clarification and sterile filtration of cell-culture lysates. The kit eliminates the centrifugation step otherwise needed for clarification.

To clarify and sterilize the sample, a diatomaceous earth (DE) was added inside the bottle-top filter and then the cell lysate was added. A vacuum was attached to the nozzle on the filter to draw the lysate through the filter.

To minimize rAAV degradation, the titers of vector genome containing AAV particles in the clarified lysate were determined by qPCR immediately after clarification using ITR-specific primers or stored at -20 °C.

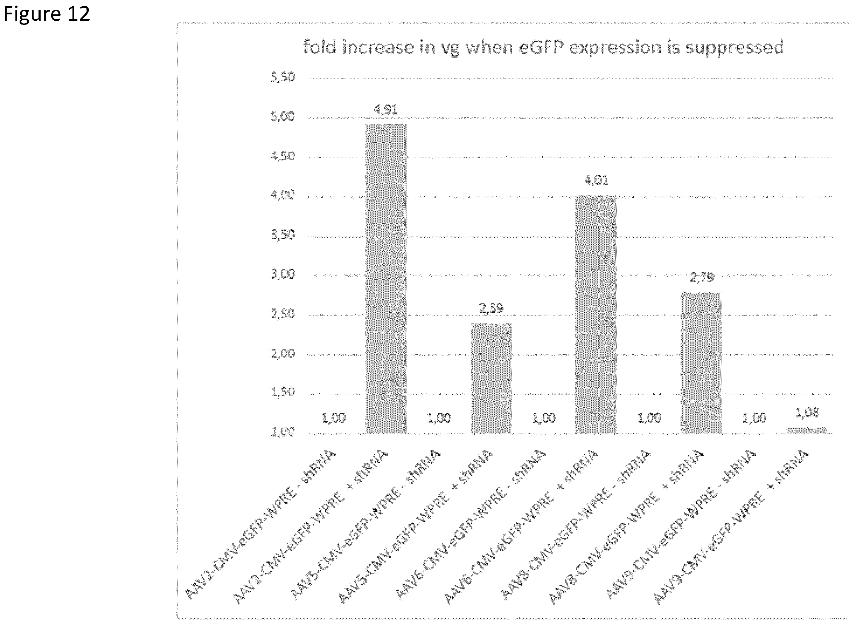

As shown in Table 3 and Fig. 12, for serotypes AAV2, AAV5, AAV6 and AAV8 tested an up to 5-fold increase in upstream productivity was obtained, as measured by vector genome titers (vg/mL) in crude lysates. The increase of productivity for AAV9 was of 1.1-fold.

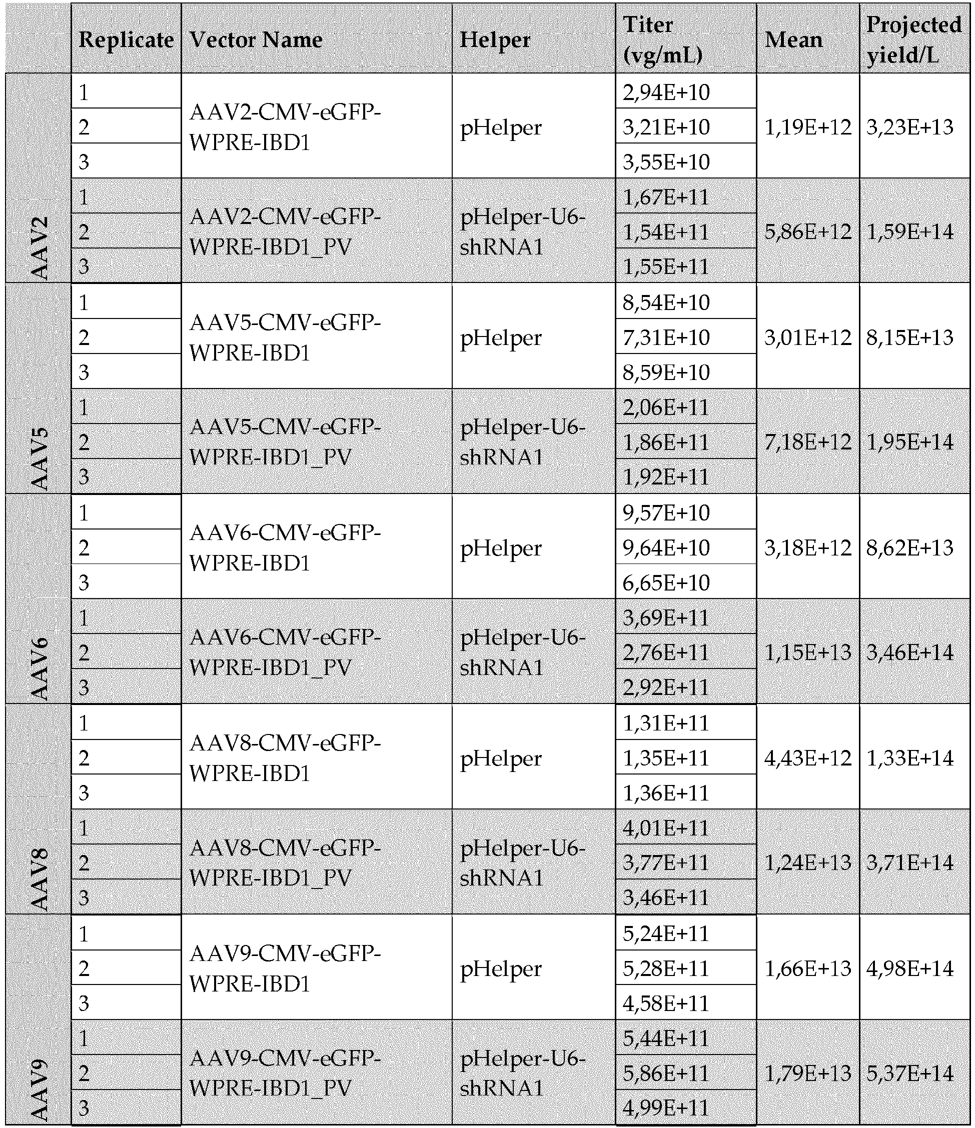

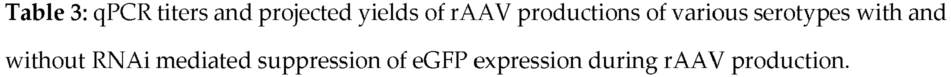

### Example 4: Suppression of cytotoxic genes during rAAV production

The effect of transgene suppression on rAAV vector yield was tested for genes with known cytotoxic properties. For this purpose, the following 3 genes were chosen.
1. Murine RIPK3: A component of the TNF receptor I complex which can induce apoptosis;
2. TRIF: Interacts with TLR3, component of the NFkb pathway, acts as an antiviral defense; and
3. eGFP-KASH fusion protein, which binds to the nuclear membrane and interferes with rAAV production.

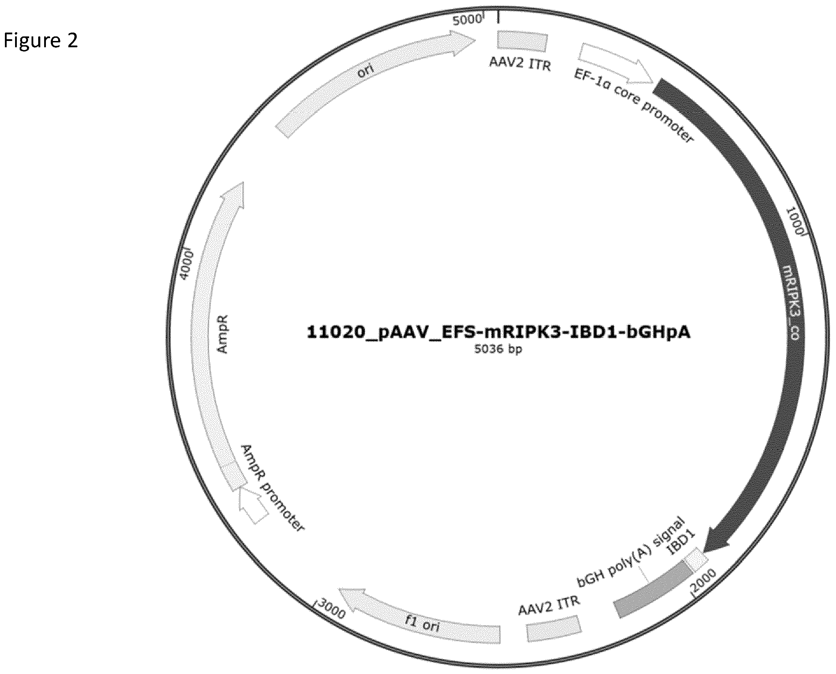

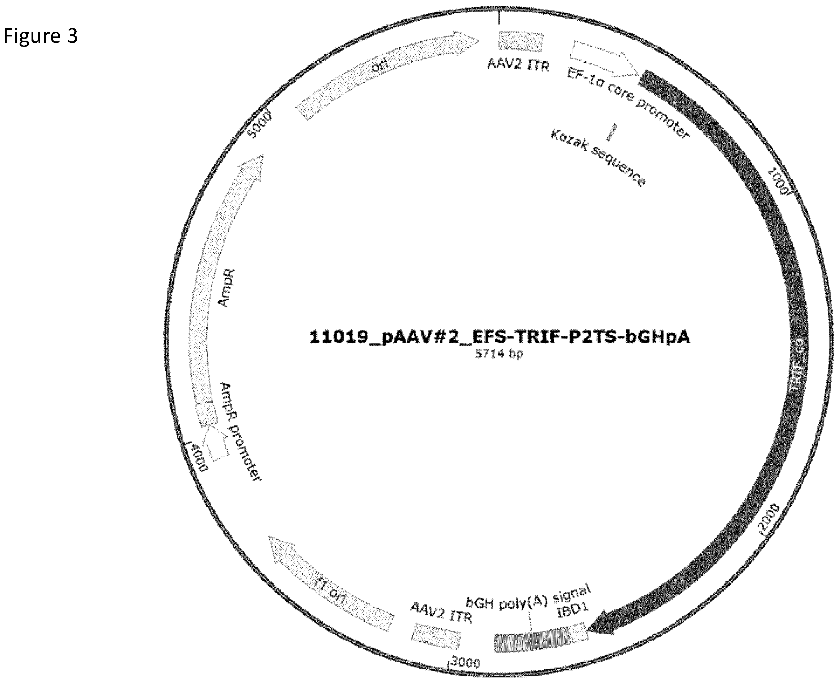

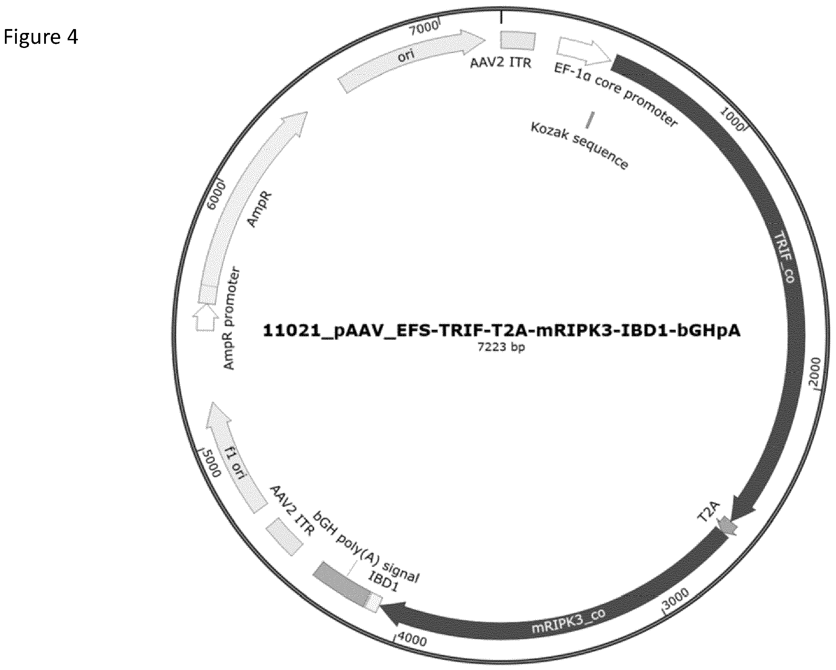

The following rAAV vector plasmids were generated:
For the generation of the transgene plasmids to express mRIPK3, TRIF and TRIF-T2a, expression cassettes EFS-mRIPK3-IBD1, EFS-TRIF-IBD1 and EFS-TRIF-T2a-mRIPK3-IBD1 were synthesized at Geneart (Regensburg) and cloned into a pDonor Plasmid. The expression cassettes of the resulting pENTR-plasmids were then transferred into pAAV-DEST via Gateway recombination to obtain plasmids pAAV-EFS-mRIPK3-IBD1 (see Fig. 2), pAAV-EFS-TRIF-IBD1 (see Fig. 3) and pAAV-EFS-TRIF-T2a-mRIPK3-IBD1 (see Fig. 4).

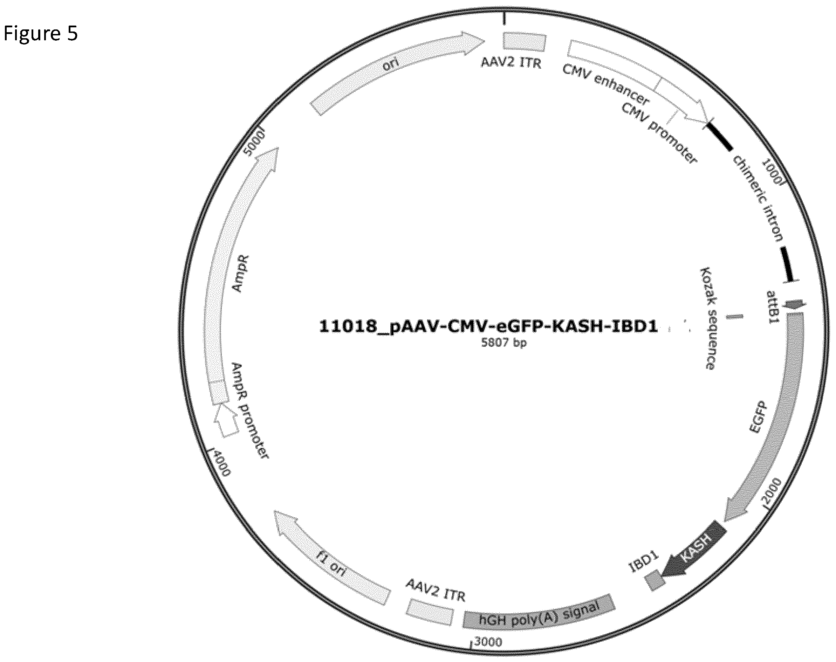

The plasmid pAAV-CMV-eGFP-KASH-IBD1 was generated by insertion of the 47 bp IBD1 fragment into the Bmt I site downstream of the eGFP-KASH stop codon of plasmid pAAV-CMV-eGFP-KASH via Gibson assembly. See Fig. 5.

The production of recombinant AAVs (rAAV) of the serotype AAV2-7m8 was performed in 30 mL scale by triple transfection of Viral Production Cells 2.0 (VPCs 2.0 from Gibco^{™}) with the transgene plasmids expressing the cytotoxic genes, the helper plasmid pHelper-U6-shRNA1 and the packaging plasmid pRep2-CAP2.7m8. rAAV productions of serotype AAV2.7m8 were then performed with and without suppression of the cytotoxic genes. The transfected combinations are listed in Table 4 below:

**Table 4: Plasmids combinations transfected into VPC cells.**

| | **Transgene plasmid** | **Helper Plasmid** | **Packaging Plasmid** | **Resulting serotype** |
|---|---|---|---|---|
| 1 | pAAV-EFS-mRIPK3-IBD1 | pHelper | pRep2-CAP2.7m8 | AAV2.7m8 |
| 2 | pAAV-EFS-mRIPK3-IBD1 | pHelper-U6-shRNA1 | pRep2-CAP2.7m8 | AAV2.7m8 |
| 3 | pAAV-EFS-TRIF-IBD1 | pHelper | pRep2-CAP2.7m8 | AAV2.7m8 |
| 4 | pAAV-EFS-TRIF-IBD1 | pHelper-U6-shRNA1 | pRep2-CAP2.7m8 | AAV2.7m8 |
| 5 | pAAV-EFS-TRIF-T2a-mRIPK3-IBD1 | pHelper | pRep2-CAP2.7m8 | AAV2.7m8 |
| 6 | pAAV-EFS-TRIF-T2a-mRIPK3-IBD1 | pHelper-U6-shRNA1 | pRep2-CAP2.7m8 | AAV2.7m8 |
| 7 | pAAV-CMV-eGFP-KASH-IBD1 | pHelper | pRep2-CAP2.7m8 | AAV2.7m8 |
| 8 | pAAV-CMV-eGFP-KASH-IBD1 | pHelper-U6-shRNA1 | pRep2-CAP2.7m8 | AAV2.7m8 |

Transfections were set up in triplicates for each condition and performed as in Example 2. The harvesting and clarifications of the rAAV particles with and without suppression of the cytotoxic genes were performed as in Example 3.

The titers of vector genome containing AAV particles in the clarified lysate were determined by qPCR after 72h from crude cell lysates.

Table 5 below shows the qPCR titers and projected yields of rAAV2.7m8 productions with and without RNAi mediated suppression of vector-encoded cytotoxic genes during productions.

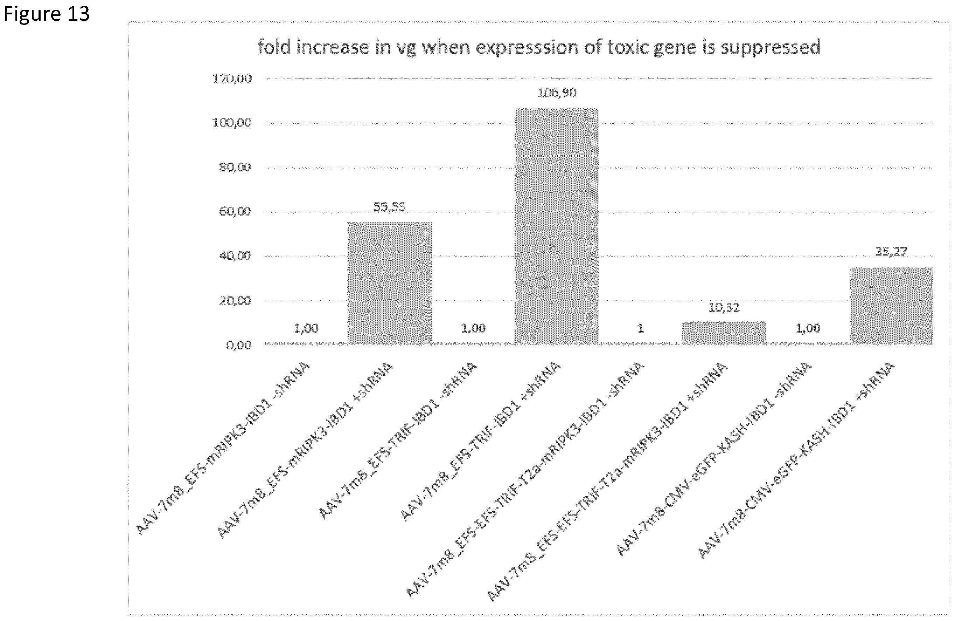

**Table 5:** qPCR titers and projected yields of rAAV productions of AAV2 with and without RNAi mediated suppression of cytotoxic genes expression during rAAV production (toxic gene 1: mRIPK3; toxic gene 2: TRIF; toxic gene 3: TRIF-T2a + mRIPK3 and toxic gene 4: KASH). As shown in Table 5 and Fig. 13, an increase in upstream productivity between around 10-fold and around 100-fold was obtained when the cytotoxic transgenes were silenced by shRNA1, as measured by vector genome titers (vg/mL) in crude lysates. In particular, a 10-fold, a 35-fold, a 55-fold and a 106-fold increase in productivity was observed for genes RIPK3+TRIF, eGFP-KASH, RIPK3 and TRIF, respectively.

### Example 5: Test of alternative shRNA or shmir sequences

A second shRNA and a shRNA within a microRNA backbone were tested to supress the expression of the cytotoxic gene mRIPK3.

The second shRNA (shRNA2) was generated for specific silencing of eGFP and comprises the sequence set out in SEQ ID NO: 3:

ACCGTACAACAGCCACAACGTCTATATCAAGAGTATAGACGTTGTGGCTGTT
GTATTTTTTG

The shaded regions of the shRNA2 are the two reverse complementary arms of the shRNA forming the
hairpin.

shMIR1 (H1-shmir) expresses an shRNA within a miRNA-155 backbone and comprises the following sequence (SEQ ID NO: 5). In the sequence below, the shaded regions are the reverse complementary sequences of the inserted shRNA and the underlined sequences are original miR-155 sequences:

CTGGAGGCTTGCTGAAGGCTGTATGCTGTGTAGTATCGGATCTTGGCCTGTTT
TGGCCACTGACTGACAGGCCAAGCCGATACTACACAG
GACACAAGGCCTGTTACTAGCACTCACATGGAACAAATGGCC

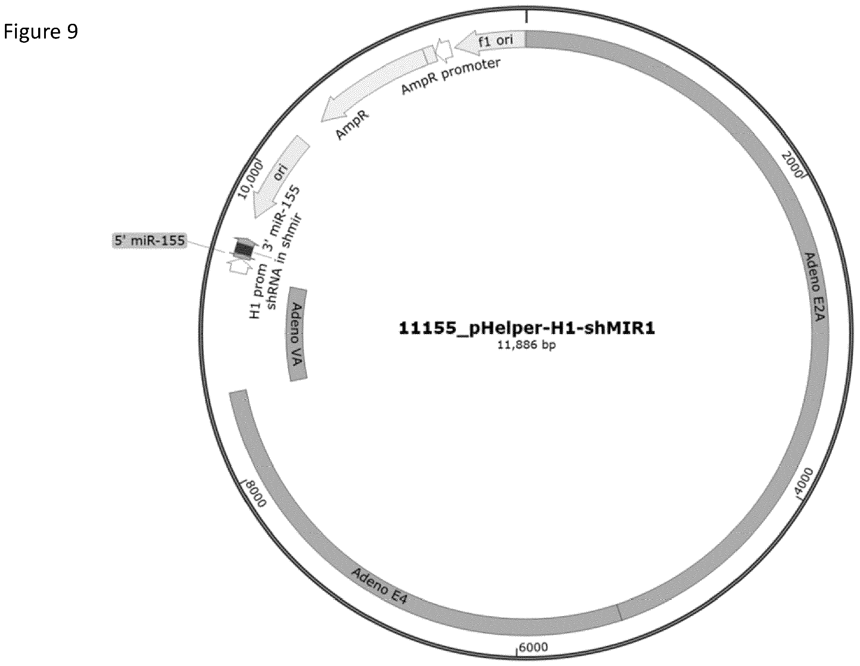

U6-shRNA2 and H1-shmir1 expression cassettes were cloned as described under Example 1 into plasmid pHelper to result in pHelper-U6-shRNA2 (see Fig. 8) and pHelper-H1-shmir1 (see Fig. 9), respectively.

Sequences of the shRNA2 and shmir target sites (IBD2 and IBD3, respectively) are set out below:
IBD2 (SEQ ID NO: 4):
   GAAGAATGTTACAACAGCCACAACGTCTATATTCAGGACAAGA
IBD3 (SEQ ID NO: 6):
   TAGGCCAAGATCCGCTACTACAATTCAGGACAAGACTAGT

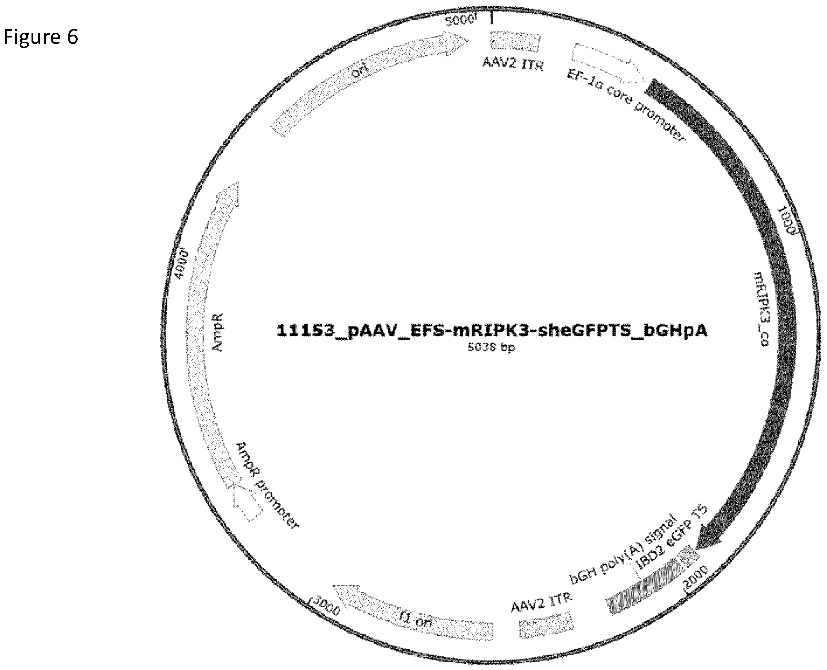

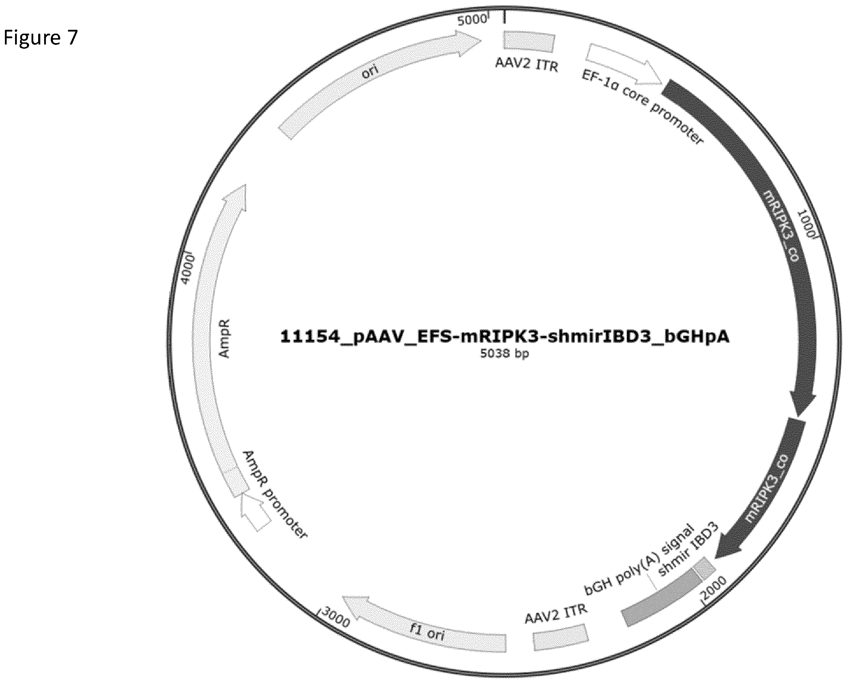

The IBD1 target site of plasmid pAAV-EFS-mRIPK3-IBD1 was replaced by the corresponding target sites IBD2 and IBD3, resulting in transgene plasmids pAAV-EFS-mRIPK3-IBD2 (see Fig. 6) and pAAV-EFS-mRIPK3-IBD3 (see Fig. 7).

rAAV productions of serotype AAV2 were then performed with and without suppression of the cytotoxic gene mRIPK3 using the following combinations of transgene (or vector plasmid), helper and packaging plasmids shown below in Table 6.

**Table 6: Plasmids combinations transfected into VPC cells.**

| | **Vector plasmid** | **Helper Plasmid** | **Packaging Plasmid** | **Resulting serotype** |
|---|---|---|---|---|
| 1 | pAAV-EFS-mRIPK3-IBD2 | pHelper | pRep2-CAP2 | AAV2 |
| 2 | pAAV-EFS-mRIPK3-IBD2 | pHelper-U6-shRNA2 | pRep2-CAP2 | AAV2 |
| 3 | pAAV-EFS-mRIPK3-IBD3 | pHelper | pRep2-CAP2 | AAV2 |
| 4 | pAAV-EFS-mRIPK3-IBD3 | pHelper-H1-shmir1 | pRep2-CAP2 | AAV2 |

Transfections were set up in triplicates for each condition and performed as in Example 2. The harvesting and clarifications of the rAAV particles with and without suppression of the cytotoxic genes were performed as in Example 3.

The titers of vector genome containing AAV particles in the clarified lysate were determined by qPCR after 72h from crude cell lysates.

Table 7 below shows the qPCR titers and projected yields of rAAV2.7m8 productions with and without RNAi mediated suppression of the vector-encoded cytotoxic gene mRIPK3 using other shRNA or shmir sequences for transgene silencing.

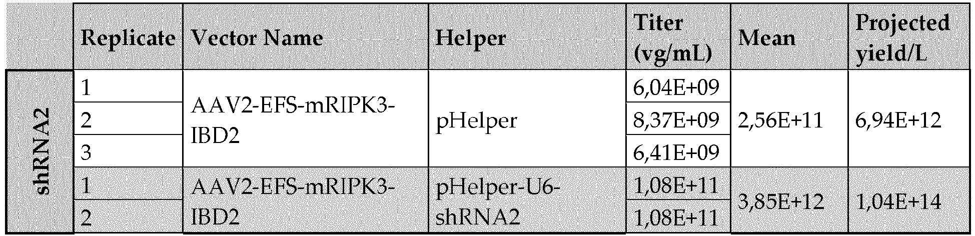

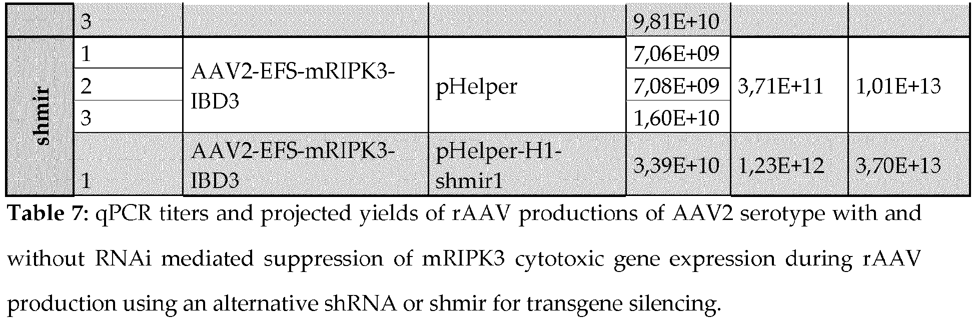

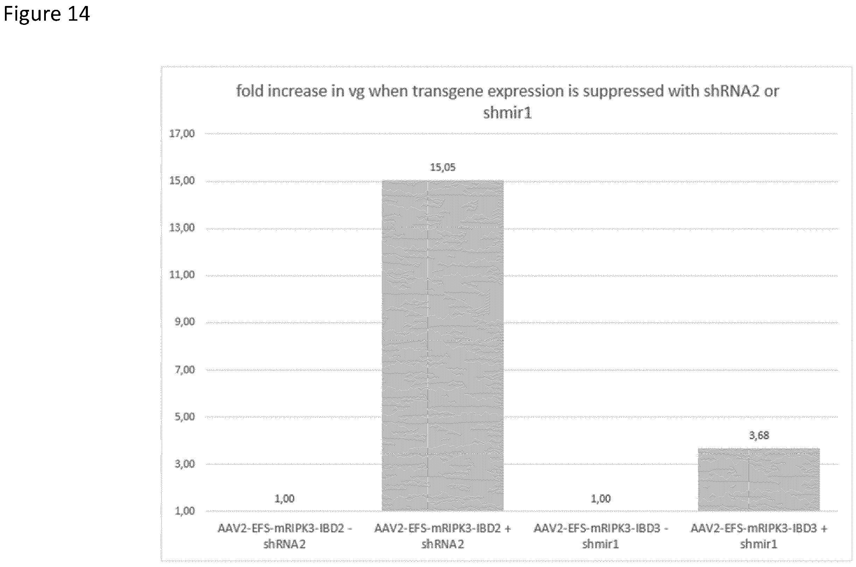

As shown in Table 7 and Fig. 14, an increase in upstream productivity of around 10-fold was obtained when the cytotoxic mRIPK3 transgene was silenced by shRNA2 and an increase of productivity of around 4-fold was obtained when the cytotoxic mRIPK3 transgene was silenced by shmir, as measured by vector genome titers (vg/mL) in crude lysates. This confirms that the suppression of cytotoxic expression can be obtained by using a variety of RNAi-mediated components.

Particular aspects and embodiments of the disclosure are set forth in the following numbered paragraphs:
1. A combination of nucleic acids for the production of adeno-associated viral (AAV) particles or retroviral particles, said combination comprising or consisting of
   (a) a first nucleic acid encoding or being an inhibitory RNA;
   (b) at least one second nucleic acid comprising helper nucleic acids necessary for production of said viral particles, and/or encoding helper proteins necessary for said production; and
   (c) a third nucleic acid comprising a binding site for said inhibitory RNA.
2. The combination of item 1, wherein said inhibitory RNA is a small inhibitory RNA, such as an shRNA, a miRNA, an siRNA or an antisense RNA.
3. The combination of item 1 or 2, wherein the third nucleic acid comprises a nucleic acid of interest.
4. The combination of any one of items 1 to 3, wherein, in case said viral particles are adeno/associated viral (AAV) particles, said third nucleic acid comprises inverted terminal repeats (ITRs), and in case said viral particles are retroviral particles, said third nucleic acid comprises long terminal repeats (LTRs), respectively.
5. The combination of item 3 or 4, wherein said nucleic acid of interest is a gene of interest, and preferably said gene of interest encodes a protein of interest or a non-coding RNA.
6. The combination of any of items 1 to 5, wherein said inhibitory RNA does not bind to the second nucleic acid(s).
7. The combination of any one of items 1 to 6, wherein
   (i) said first nucleic acid and said at least one second nucleic acid are comprised in a first plasmid and said third nucleic acid is comprised in a second plasmid;
   (ii) said first nucleic acid and said third nucleic acid are comprised in a first plasmid and said at least one second nucleic acid is comprised in at least one second plasmid;
   (iii) said first nucleic acid is comprised in a first plasmid, said second nucleic acid is comprised in at least one second plasmid and said third nucleic acid is comprised in a third plasmid;
   (iv) said first nucleic acid, said at least one second nucleic acid and said third nucleic acid are comprised in the same plasmid; or
   (v) said first nucleic acid is comprised in a first plasmid, and said at least one second nucleic acid and said third nucleic acid are comprised in a second plasmid.
8. The combination of any of the preceding items, wherein said first nucleic acid encodes said inhibitory RNA and comprises an RNA polymerase III promoter; a small RNA-expressing promoter, such as a U6 promoter or H1 promoter; an RNA polymerase II promoter; and/or a promoter with activity in eukaryotic cells such as CMV promoter.
9. The combination of any one of items 5 to 8, wherein said inhibitory RNA inhibits transcription of said nucleic acid of interest and/or expression of said protein of interest.
10. The combination of any one of items 1 to 9, wherein said binding site is located in the nucleic acid of interest, such as in an untranslated region (UTR) of said nucleic acid of interest.
11. The combination of item 10, wherein said binding site is a sub-sequence of said nucleic acid of interest.
12. The combination of item 10, wherein said binding site is unrelated to said nucleic acid of interest.
13. The combination of any one of the preceding items, wherein
   (a) said particles are AAV particles and said helper proteins or helper nucleic acids comprise Rep, Cap, E2A, E4, VA, and E1; or
   (b) said particles are retroviral particles and said helper proteins or helper nucleic acids comprise gag, pol, env, and rev.
14. The combination of any one of the preceding items, wherein said combination does not comprise further means suitable for inhibiting transcription or translation of a gene of interest, in particular not components of the Tet system and/or the tamoxifen inducible Cre system.
15. The combination of any one of items 2 to 14, wherein said inhibitory RNA is an shRNA and preferably comprises
   a. the sequence of SEQ ID NO: 1 or a sequence at least 80%, at least 85%, at least 90%, at least 95%, at least 98%, at least 99%, at least 99.5%, or 100% identical to the sequence of SEQ ID NO: 1; or
   b. the sequence of SEQ ID NO: 3 or a sequence at least 80%, at least 85%, at least 90%, at least 95%, at least 98%, at least 99%, at least 99.5%, or 100% identical to the sequence of SEQ ID NO: 3, respectively.
16. The combination of item 15, wherein the binding site of said shRNA comprises
   a. the sequence of SEQ ID NO: 2 or a sequence at least 80%, at least 85%, at least 90%, at least 95%, at least 98%, at least 99%, at least 99.5%, or 100% identical to the sequence of SEQ ID NO: 2; or
   b. SEQ ID NO: 4 or a sequence at least 80%, at least 85%, at least 90%, at least 95%, at least 98%, at least 99%, at least 99.5%, or 100% identical to the sequence of SEQ ID NO: 4, respectively.
17. The combination of any one of items 2 to 14, wherein said inhibitory RNA is a miRNA and preferably comprises the sequence of SEQ ID NO: 5 or a sequence at least 80%, at least 85%, at least 90%, at least 95%, at least 98%, at least 99%, at least 99.5%, or 100% identical to the sequence of SEQ ID NO: 5.
18. The combination of item 17 wherein the binding site of said miRNA has the sequence of SEQ ID NO: 6 or a sequence at least 80%, at least 85%, at least 90%, at least 95%, at least 98%, at least 99%, at least 99.5%, or 100% identical to the sequence of SEQ ID NO: 6.
19. Use of the combination of any of items 1 to 18 for transfection of a cell.
20. The use of item 19, wherein said use is in vitro or ex vivo and/or said cell is a cell in culture.
21. A method of transfecting a cell, said method comprising bringing a cell into contact with the components of the combination of any one of items 1 to 18.
22. The method of item 21, wherein said transfecting is
   (a) transient with regard to said first, said at least one second and said third nucleic acid,
   (b) stable with regard to said first nucleic acid and/or at least one of the at least one second nucleic acids, and transient as regards the remainder of nucleic acids, or
   (c) stable with regards to the third nucleic acid, and optionally transient as regards the first and second nucleic acids.
23. The method of item 21 or 22, wherein said method is performed in vitro or ex vivo.
24. A cell
   (a) obtained by the method of any one of items 21 to 23; and/or
   (b) comprising the components of the combination of any one of items 1 to 18.
25. The cell of item 24, wherein
   (a) the first nucleic acid and/or at least one of the at least one second nucleic acids is/are integrated into the genome of said cell; and/or
   (b) the third nucleic acid is integrated into the genome of said cell.
26. The cell of item 24 or 25, wherein said cell is of mammalian or insect origin, wherein, for example, said cell of mammalian origin is of human origin, such as HEK-293, HeLa, and CAPO, or said cell is of insect origin is Sf9.
27. A method of producing viral particles carrying a nucleic acid of interest, said method comprising
   (a) bringing a cell into contact with the components of the combination of any one of items 3 to 18; and
   (b) allowing formation of said viral particles to occur;
   wherein preferably said method is an in vitro or ex vivo method and/or said cell is a cell in culture.
28. The method of item 27, wherein the third nucleic acid of said combination comprises a gene of interest which encodes a protein of interest, and wherein
   (a) said protein is not produced or is not substantially produced; and/or
   (b) the production of said viral particles is increased as compared to the absence of said first nucleic acid.
29. The combination of any one of items 3 to 18, the use of item 19 or 20, the method of any one of items 21 to 23 to the extent said items refer back to any one of items 3 to 18, the cell of any one of items 24 to 26 to the extent a nucleic acid of interest is present, or the method of any one of items 27 to 28, wherein said nucleic acid of interest is or encodes a vaccine, is a therapeutic gene or encodes a therapeutic protein, is a diagnostic gene, or encodes a protein capable of binding to a cognate binding partner.
30. The combination, use, cell or method of item 29, wherein said therapeutic gene or said therapeutic protein complements a genetic disorder and/or a disorder related to under-expression of the naturally occurring counterpart of said gene or protein; encodes or is a chimeric antigen receptor (CAR); encodes or is a base editor; or said cognate binding partner is a biomolecule of diagnostic or analytical interest, said biomolecule preferably being a protein.

## Claims

1. A combination of nucleic acids for the production of adeno-associated viral (AAV) particles or retroviral particles, said combination comprising or consisting of
a. a first nucleic acid encoding or being an inhibitory RNA;
b. at least one second nucleic acid comprising helper nucleic acids necessary for production of said viral particles, and/or encoding helper proteins necessary for said production; and
c. a third nucleic acid comprising a binding site for said inhibitory RNA.

2. The combination of claim 1, wherein said inhibitory RNA is a small inhibitory RNA, preferably an shRNA, a miRNA, an siRNA or an antisense RNA.

3. The combination of claim 1 or 2, wherein the third nucleic acid comprises a nucleic acid of interest.

4. The combination of any one of claims 1 to 3, wherein, in case said viral particles are AAV particles, said third nucleic acid comprises inverted terminal repeats (ITRs), and in case said viral particles are retroviral particles, said third nucleic acid comprises long terminal repeats (LTRs), respectively.

5. The combination of claim 3 or 4, wherein said nucleic acid of interest is a gene of interest, and preferably encodes a protein of interest.

6. The combination of any of claims 1 to 5, wherein said inhibitory RNA does not bind to the second nucleic acid(s).

7. The combination of any one of claims 1 to 6, wherein
(i) said first nucleic acid and said at least one second nucleic acid are comprised in a first plasmid and said third nucleic acid is comprised in a second plasmid;
(ii) said first nucleic acid and said third nucleic acid are comprised in a first plasmid and said at least one second nucleic acid is comprised in at least one second plasmid;
(iii) said first nucleic acid is comprised in a first plasmid, said second nucleic acid is comprised in at least one second plasmid and said third nucleic acid is comprised in a third plasmid;
(iv) said first nucleic acid, said at least one second nucleic acid and said third nucleic acid are comprised in the same plasmid; or
(v) said first nucleic acid is comprised in a first plasmid, and said at least one second nucleic acid and said third nucleic acid are comprised in a second plasmid.

8. The combination of any one of claims 5 to 7, wherein said inhibitory RNA inhibits transcription of said nucleic acid of interest and/or expression of said protein of interest.

9. The combination of any one of claims 1 to 8, wherein said binding site is located in the nucleic acid of interest, preferably in an untranslated region (UTR) of said nucleic acid of interest.

10. The combination of claim 9, wherein said binding site is a sub-sequence of said nucleic acid of interest or is unrelated to said nucleic acid of interest.

11. The combination of any one of claims 1 to 10, wherein said combination does not comprise further means suitable for inhibiting transcription or translation of a gene of interest, in particular not components of the Tet system and/or the tamoxifen inducible Cre system.

12. Use of the combination of any of claims 1 to 11 for transfection of a cell.

13. A method of transfecting a cell, said method comprising bringing a cell into contact with the components of the combination of any one of claims 1 to 11.

14. A cell
a. obtained by the method of claim 13; and/or
b. comprising the components of the combination of any one of claims 1 to 11.

15. A method of producing viral particles carrying a nucleic acid of interest, said method comprising
a. bringing a cell into contact with the components of the combination of any one of claims 3 to 11; and
b. allowing formation of said viral particles to occur;
wherein preferably said method is an in vitro or ex vivo method and/or said cell is a cell in culture.
